Europäisches Patentamt

European Patent Office

Office européen des brevets

(11) Publication number: **0 303 472**
**A1**

## EUROPEAN PATENT APPLICATION

(21) Application number: 88307411.4

(22) Date of filing: 10.08.88

(51) Int. Cl.⁴: **A 01 G 7/00**

(30) Priority: 12.08.87 GB 8719082
08.10.87 GB 8723615

(43) Date of publication of application:
15.02.89 Bulletin 89/07

(84) Designated Contracting States:
AT BE CH DE ES FR GB GR IT LI LU NL SE

(71) Applicant: Albright & Wilson Limited
210-222 Hagley Road West
Oldbury Warley West Midlands B68 0NN (GB)

(72) Inventor: Mavituna, Ferda
15 Bodmin Drive Bramhall
Stockport Cheshire SK7 2HX (GB)

Williams, Paul David
46 Gladstone Street
Glossop Derbyshire (GB)

Hudson, Eric James
Loscar 9 Woodlands Avenue Hillcrest
Whitehaven Cumbria CA28 6TF (GB)

Law, David
9 Bradshaw Road Marple
Stockport Cheshire SK6 6PF (GB)

(74) Representative: Savidge, Roger Gordon Madgwick et al
c/o Albright & Wilson Limited 1 Knightsbridge Green
London SW1X 7QD (GB)

(54) Plant propagation.

(57) Plants are micropropagated by a process which comprises forming an aqueous suspension of comminuted plant material, separating from the suspension particles capable of regeneration into plants (propagules), directing the propagules to a plurality of discrete growing sites on a supporting medium supplied with plant growth nutrients, growing the propagules and separating the growing sites and the plantlets or plants grown thereon.

The propagules are separated from the suspension by detection of their particle size or morphology e.g. by means of a fluidic switch.

The support material is preferably cellulosic e.g. paper pulp or other cellulosic fibrous material.

EP 0 303 472 A1

## Description

### 'PLANT PROPAGATION'

This invention relates to plant propagation and more particularly to micropropagation, the process of large scale multiplication of individual plants from small parts, tissues, or cells grown aseptically in culture.

In conventional micropropagation the growing tip (meristem) of the plant is excised and grown on a nutrient medium into a clump containing multiple shoots, which have to be separated one from another and subcultured on fresh medium. The separated shoots are grown further and may undergo one or more cycles of reproduction in order to produce large numbers of plantlets. The plantlets are then transferred to a rooting medium, separated and potted for further growth into mature plants. The whole process is highly labour intensive since each step is performed by hand. Relatively small numbers of plants are prepared from each original plant in a single operation because of the limited amount of meristem tissue available from each plant and the belief that if the tissue is subdivided into fragments smaller than a certain critical size, the resultant particles cannot be efficiently regenerated into plants.

EP-A-0132414 (Milouda) discloses a method which is described as suitable for semi-automated micropropagation, wherein meristems produced by conventional manual culturing and excision are comminuted in a blender and classified to obtain optimum sized particles for distribution to culture vessels. The foregoing method involves relatively little saving of labour since both the preparation of the meristem tissue prior to comminution and the subsequent separation and potting out of the plantlets must be carried out manually.

It is the object of the present invention to devise a less laborious and more direct method of micropropagation from the initial plant material and one which is more readily amenable to automation.

According to this invention a method of plant micropropagation comprises forming an aqueous suspension of comminuted plant material, separating from the suspension particles capable of regeneration into plants (propagules) and directing them to a plurality of discrete growing sites on a supporting medium supplied with plant growth nutrients, growing the propagules and subsequently separating the growing sites and the plantlets or plants grown thereon.

The method of this invention begins with whole plants or with plant material recycled from later stages in the process, as will be described hereinafter. The initial plant material is cleaned, disinfected and sterilised to remove all traces of contamination prior to comminution. The plants may be comminuted by maceration in a suitable high shear homogeniser, for example, as described in our British Patent Application 2162537. In the initial homogenised mass the total number of meristematic and other particles capable of regeneration can give rise to a very large number of final plants but if desired the comminuted cells may optionally be cultured in suspension, e.g. in a growth promoting liquid nutrient medium, for from 1 to 50, e.g. 2 to 30, preferably 4 to 15 days to provide an increased concentration of viable particles.

The homogenised mass, either in its initial form or after the cultivation stage just described, contains material extending over a large range of particle size and it is necessary to separate from the suspension particles capable of regeneration into plants i.e. propagules. It is desirable to carry out a first or crude separation to remove coarse particles. Preferably the suspended particles are sieved to provide particles each of a size sufficient to produce a single plant. Preferably each particle comprises from 1 to 2000, e.g. from 2 to 1000 cells. Typically this requires a particle size up to 2 mm, e.g. from 0,5 to 1 mm. It is then desirable to achieve a more refined separation to separate propagules for the inoculation and growth stages of the present invention. One method of separating the desired fraction is to a set up a flowing stream of the homogenised mass and to pass the stream through a detector device capable of detecting particles on the basis of an appropriate parameter such as particle size or morphology, for example, using image analysis techniques. For instance a stream of the suspension may be passed through a particle detector such as a light beam illuminating a photoelectric cell, which is adapted to divert any particle of sufficient size to the inoculator. Diversion of the particles may for instance be effected by means of a fluidic switch, e.g. of the Coanda effect type wherein jets of gas such as compressed air or liquid temporarily divert a stream of liquid containing the suspended particles into a channel leading to the inoculator whenever a particle of appropriate size is detected.

After the separation of the desired particles they are inoculated on to a solid, gelatinous or pasty support medium. The medium may be a conventional agar gel or compost, a foamed polymer or woven fabric support or more conveniently a web of cellulosic fibre such as a sheet of unsized paper or cardboard. A particularly preferred support medium is one composed of pulped cellulosic fibres such as pulped filter paper preferably pulped secondary fibre as recycled newsprint. Paper pulp, in addition to offering a substantial cost advantage over the conventionally used agar gel, tends to stimulate excellent root growth.

Preferably, the support medium is disposed in a propagator comprising a container such as a tray, dish or similar vessel. The container is preferably formed from a water-impermeable material such as plastic or metal which is capable of chemical or thermal sterilisation. Preferably the container is designed to permit easy removal of liquid nutrients and replacement by fresh or different media. The base may optionally be perforated to permit drainage but is preferably designed to drain from a single drainage point. The pulp is preferably impregnated with an aqueous solution of plant nutrients and sterilised, e.g. by autoclaving. The inside of the container may be subdivided, e.g. by upright longitudinal and/or transverse partitions into channels or compartments, e.g. as in the ice tray of a refrigerator, in order to provide discrete propagation sites. Preferably the partitions are separable from the container in order to facilitate separation of the pulp in each compartment. Alternatively, separate sites may be defined by the interstices of a perforated plate, mesh or grid on the surface of the pulp.

Where a solid sheet of support medium is employed the propagation sites may be defined by corrugations or depressions in the sheet. The sheet may be a continuous sheet which is passed continuously or intermittently through an inoculation zone at which it is sequentially inoculated at a plurality of sites and then through the propagation zone, the sites being finally separated by cutting or otherwise dividing the sheet. Alternatively or additionally the sheet may be divided after inoculation and prior to entering the propagation zone, into portions each comprising one or more sites. The sheet may initially be a discontinuous sheet comprising a plurality of separate portions each comprising one or more sites.

The inoculator may comprise a plurality of orifices adapted simultaneously to inoculate each of a set of discrete sites, subsequent sets of sites being inoculated at discrete intervals as the layer of supporting medium passes through the inoculator. However a particularly convenient method of inoculation is by means of a cartesian robot. The inoculated support medium is then conveyed to a growth (propagation) zone, or a series of such zones, in which the propagules develop into plantlets at their respective spaced positions on the support. The ordered and regular location of the potential plants permits of easy removal of the developed plantlets by mechanised or automated separator devices and distribution to their final location.

The micropropagation process of the present invention, as outlined above, is capable of further modification and development in order to realise to the maximum the growth potential of the homogenate formed in the initial stage of operation. For example, explants or comminuted plant tissue may be suspended in an aqueous nutrient medium adapted to promote plant development, e.g. by inclusion of appropriate auxins and cytokinins. We have discovered that plantlets develop well in liquid suspension culture from meristem or homogenised plant tissue and may develop an increased number of fresh growing points compared to plantlets propagated in a solid or gel support medium in the conventional manner. Although, plantlets grown in liquid suspension culture may adopt an atypical habit which is unsuitable for direct potting on, they may be highly suitable as source of fresh meristem tissue for further micropropagation. Preferably plantlets grown in liquid suspension culture are homogenised to provide fresh tissue for micropropagation or planted out on a solid, gelatinous or pasty support to provide fresh shoots for excision or mowing, followed by conventional micropropagation on a solid, pasty or gel support or by micropropagation according to any of the novel methods of the invention.

Again, instead of allowing the plantlets grown at the spaced-apart discrete sites to proceed to develop as final plants they may be used as a source of additional growth material by excision of tips or parts of stems for recycling to the homogenisation stage. For example, a mowing device may be passed over the plantlets at a height adapted to excise the growing tips. The mower may comprise rotating or reciprocating blades, and is preferably provided with means for collecting the excised tips, such as a collecting box into which the explants are swept by the action of the blades and/or preferably a suction line for collecting and transporting the explants to a subsequent stage in the propagation process. Preferably the unmown portions of the mown plantlets are allowed to regrow, producing an increased yield of new shoots, and are remown at intervals. Optionally the height of the mower may be adjusted prior to each mowing. Conveniently, but not essentially, the plantlets for mowing may be grown in a regular spaced array formed by inoculation of a solid, gelatinous or pasty support as hereinafter described.

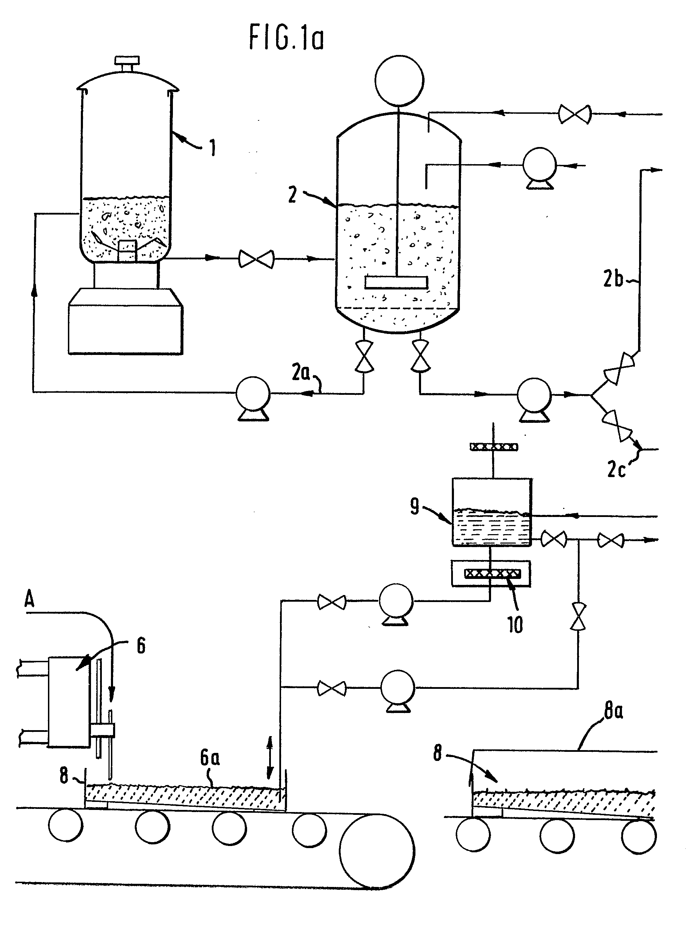

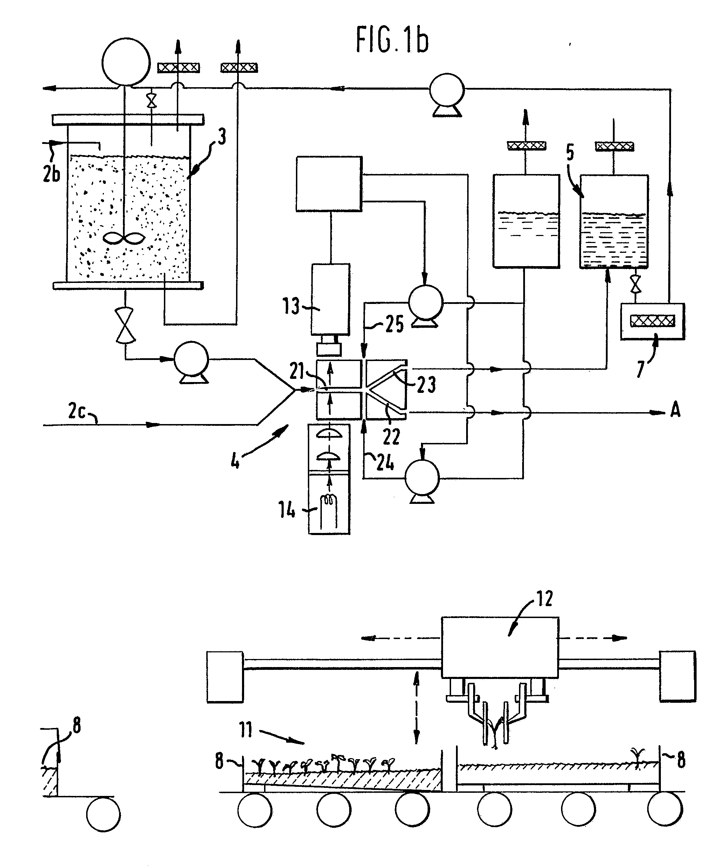

A micropropagation system according to this invention is illustrated by way of example in the accompanying composite drawings, figures 1a and 1b which are to be read together. The major components are described below, the remainder being indicated by conventional symbols for fluid supply lines, pumps, valves etc.

The system comprises a homogeniser 1, which is a commercial blender adapted to comminute plant tissue, and a separator 2 to receive comminuted tissue from the homogeniser 1 and provide a first crude separation of regenerable particles from cell debris and coarse fragments by decantation and/or filtration. Coarse particles are returned to the homogeniser through line 2a and the fine particle fraction is passed either through line 2b to a culture vessel (fermenter) 3 or through line 2c to a fluidic switch 4. The fermenter 3 maintains selected particles from the separator 2 in suspension in growth promoting nutrient medium, e.g. by means of air agitation and develops or multiplies propagules in the initial homogenate. The resulting material is then transferred to the fluidic switch 4 through line 2d.

The fluidic switch 4 comprises an optical detection system 13, 14 designed to detect regenerable particles flowing in line 21 and to divert them through lower line 22 to an inoculator (indicated at A in figures 1a and 1b) and to pass residual medium through line 23 to a reservoir 5. The inoculator, indicated by reference numeral 6 in figure 1b, is a cartesian robot programmed to deposit inoculum at discrete spaced growing sites on a suitable support medium.

The apparatus comprises a steriliser 7 adapted to sterilise, e.g. by heating under pressure, and preferably filter, nutrient medium from the reservoir 5 and recycle sterile medium to either or both of the homogeniser 1 and the fermenter 3.

The support material comprises a layer of paper pulp 6a disposed in a propagator tray 8 and supplied with nutrient medium, under controlled conditions of temperature and illumination, e.g. by total or partial immersion, spraying, trickling or other means of irrigation from a reservoir 9 via a steriliser 10. The propagator 8 has an integral sloping base leading down to a drainage point for removal of spent nutrient. The propgator 8 is covered with a lid 8a throughout the growing period. The apparatus comprises a plantlet separator 11, including an imaging robot 12, and which is adapted to separate and preferably grade plantlets growing at the inoculated sites in the solid support medium recovered from the propagator 8, and optionally to recycle some of the plantlets to the homogeniser 1.

The apparatus is adapted for sterile operation, being protected from contact with unfiltered air, e.g. by enclosure within a structure maintained under positive air pressure. Means are preferably provided to permit fumigation of the equipment and/or thermal or chemical sterilisation of all vessels and pipework. Preferably all operations are automated and performed continuously or semi-continuously.

In operation, live sterilised plant tissue is fed to the homogeniser 1 together with nutrient medium from the steriliser 7 and comminuted to provide a major proportion of particles in the range 0.5 to 1mm. The suspension is passed to the separator 2, where the 0.5 mm to 1 mm particles are separated from cell debris and larger fragments by decantation and/or filtration and pumped to the fermenter 3. The fermenter 3 is supplied with nutrient medium from the steriliser 7, in which the particles are suspended by bubbling sterilised air therethrough. Liquid medium from the fermenter passes through the line 21 in fluidic switch 4. Initially the liquid stream is caused by the Coanda effect to pass along the line 23, by passing a pulse of sterile compressed air through the jet 24. Whenever a particle of greater than 0.5 mm passes through a transparent portion of the line 21, the interruption of a beam of light falling on a photocell from a light source triggers a pulse of air from the jet 25 which diverts the portion of the liquid stream containing the particle along the line 22 to the inoculator 6. Immediately thereafter a pulse from the jet 24 switches the stream back into the line 23.

A plurality of propagator trays 8 each divided by a separable partition grid into a plurality of open-topped compartments filled with sterile pulped newsprint impregnated with an aqueous propagating medium, or else a plurality of sheets of stiff cardboard or other fibrous or porous, water permeable support medium each formed with an array of indentations in the upper surface thereof, are fed sequentially to the inoculator 6 which inoculates each compartment or indentation with the quantity of liquid corresponding to that diverted to the inoculator 6 by the switch 4 each time the latter switches.

Nutrient medium returned to the reservoir 5 is replenished with nutrients and growth promoting chemicals favouring rapid cell growth and division in suspension culture, and passed to the steriliser 7 to be filtered, sterilised and recycled to the homogeniser 1 and the fermenter 3.

Inoculated trays or sheets of support medium 6a are supplied with liquid regeneration promoting medium, e.g. one containing a relatively high proportion of cytokynins. Alternatively and preferably the trays or sheets may be stacked and sprayed or otherwise irrigated with the liquid medium. The sheets of support medium are conveniently subjected to constant, or preferably diurnally fluctuating, illumination and temperature.

Optionally the propagation may be carried out in stages, e.g. in separate zones in which the temperature condition, and/or the composition of the liquid nutrient or compost may be adjusted to suit different stages in the regeneration and growth of plantlets at the inoculated sites. In the latter case a separate reservoir 9 may be provided for each stage.

The propagator and growing plantlets are then passed to the separator 11 for separation, grading and potting up. The plantlets may be scanned by imaging means such as a robot or remote controlled camera and the image monitored by a computer or human observer to identify plants of any particular quality. Instructions may be given to select or reject a plantlet at any specified site in the array. The sites may be separated, e.g. by means of blades severing sheets of support medium in order to separate each plantlet from its neighbours. A part of the plantlets may be recycled to the homogeniser 1 and the remainder potted out.

It will be appreciated that the present invention avoids the need for conventional explant-manipulation and -excision measures and is capable of practical embodiment in many ways, as summarised hereinafter.

According to a first embodiment a method for propagating plants comprises forming a suspension of live, comminuted plant tissue in an aqueous medium, inoculating a water-permeable, solid, gelatinous or pasty supporting medium at a plurality of discrete sites with encugh of said suspension at each site to provide a quantity of plant tissue capable of propagating a plant, contacting said supporting medium with aqueous nutrient medium adapted to promote propagation of plants from the tissue and automatically separating the plants so formed.

According to another embodiment a method for propagating plants comprises sequentially inoculating a continuous or discontinuous layer of a water-permeable supporting medium with an aqueous suspension of live comminuted plant tissue at a plurality of sites, passing said layer through a propagation zone in which it is contacted with aqueous nutrient medium under conditions favouring propagation of plants from the tissue, for a period sufficient to permit propagation to occur and subsequently dividing said layer so as to separate said sites.

According to a third embodiment this invention includes the use of a cellulosic pulp as a plant propagation medium. The invention also comprises a layer of cellulosic pulp impregnated with an aqueous solution of plant nutrients adapted to promote plant propagation, a method of propagation comprising inoculating such a layer with propagatable plant tissue and an inoculated bed of cellulosic pulp having plantlets growing therein.

According to a fourth embodiment the invention comprises a method of plant propagation wherein a plurality of plantlets in a propagating medium are mown by passing one or more rotating or reciprocating blades over said plantlets at a height adapted to remove the growing tips of the shoots, collecting said tips and contacting said tips with fresh propagating medium.

According to a fifth embodiment the invention comprises a method of plant propagation which comprises forming a suspension of live, comminuted or excised plant tissue in a liquid nutrient containing medium adapted to promote the development of plantlets therein, maintaining said tissue in suspension in said liquid medium until plantlets have developed and transferring said plantlets from said medium to a solid, gelatinous or pasty propagating medium.

## Claims

1. A method of plant micropropagation which comprises forming an aqueous suspension of comminuted plant material, separating from the suspension particles capable of regeneration into plants (propagules), directing the propagules to a plurality of discrete growing sites on a supporting medium supplied with plant growth nutrients, growing the propagules and separating the growing sites and the plantlets or plants grown thereon.

2. A method according to claim 1, in which the propagules are separated from the suspension by detection of their particle size or morphology.

3. A method according to claim 2, in which separation is achieved by means of a fluidic switch.

4. A method according to claim 1, 2 or 3, in which the separation of propagules is preceded by a preliminary separation of plant debris and other coarse material.

5. A method according to any of claims 1 to 4, in which the plant material is subjected to a stage of suspension culture prior to final separation of the propagules.

6. A method according to to any of the preceding claims, in which the support material comprises cellulosic material.

7. A method according to claim 6, in which the cellulosic material is paper pulp or other cellulosic fibrous material.

8. A method according to any of the preceding claims, in which the supporting medium is contained in a container which permits drainage of liquid medium and addition of fresh or different medium.

9. A method according to any of the preceding claims, in which the location of the growing sites is so ordered as to permit separation of the final plantlets or plants by mechanised and preferably automated procedures.

10. Micropropagated plants whenever produced by a method according to any of the preceding claims.

11. A plant propagation system comprising means for comminuting plant material into an aqueous suspension, means for separating propagules therefrom, optional means for subjecting the propagules to multiplication before or after their separation, distributing means for inoculating a support growth medium with propagules at regularly spaced locations, a growth chamber for growing the propagules into plants, and means for separating the grown plants or plantlets.

# FIG.1a

# FIG.1b

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | EP-A-0 232 628 (IND. PLANT BIOTEC INDUSTRIES)<br>* Page 9, last paragraph – page 10, paragraph 1; page 18, paragraph 1 – page 28, paragraph 3; figures 1-14 * & AU-D-67110/87 | 1,5,8-11 | A 01 G 7/00 |
| Y | | 4,6,7 | |
| D,Y | EP-A-0 132 414 (MILOUDA)<br>* Abstract * | 4 | |
| Y | EP-A-0 147 349 (GARNIER)<br>* Page 1, lines 1-3; claim 1 * | 6,7 | |
| A | WO-A-8 606 576 (SCHONSTEIN)<br>* Page 7, last paragraph – page 11, paragraph 1; figure 1 * | 2 | |
| A,P | WO-A-8 804 520 (TWYFORD PLANT LABORATORIES)<br>* Page 20, line 21 – page 23, line 15; figure 6 * | 1 | TECHNICAL FIELDS SEARCHED (Int. Cl.4) |
| A | US-A-3 995 662 (FITZGERALD)<br>* Whole document * | 3 | A 01 G<br>F 15 C |

The present search report has been drawn up for all claims

| Place of search | Date of completion of the search | Examiner |
|---|---|---|
| THE HAGUE | 18-11-1988 | HERYGERS J.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................

& : member of the same patent family, corresponding document

EPO FORM 1503 03.82 (P0401)